(19) Europäisches Patentamt
European Patent Office
Office européen des brevets

(11) Veröffentlichungsnummer: **0 108 351**
**B1**

(12) # EUROPÄISCHE PATENTSCHRIFT

(45) Veröffentlichungstag der Patentschrift: **22.07.87**

(21) Anmeldenummer: **83110778.4**

(22) Anmeldetag: **28.10.83**

(51) Int. Cl.[4]: **C 07 C 103/00**, C 07 C 103/30, C 07 C 143/86, C 07 C 125/065, C 07 C 143/82, C 07 C 103/167, C 07 C 109/06, C 07 C 109/08, C 07 C 109/10, C 07 C 145/00, C 07 C 121/75

(54) **Verwendung substituierter Malonsäurederivate als Schädlingsbekämpfungsmittel.**

(30) Priorität: **10.11.82 DE 3241512**
**10.03.83 DE 3308462**

(43) Veröffentlichungstag der Anmeldung: **16.05.84 Patentblatt 84/20**

(45) Bekanntmachung des Hinweises auf die Patenterteilung: **22.07.87 Patentblatt 87/30**

(84) Benannte Vertragsstaaten: **AT BE CH DE FR GB IT LI NL SE**

(56) Entgegenhaltungen:
**EP-A-0 076 957**
**EP-A-0 076 985**
**US-A-3 253 019**

**MONATSHEFTE FÜR CHEMIE, Band 89, 1958, WIEN (AT), K. HOHENLOHE-OEHRINGEN: "Hydrierende Cyclisierung von Azidoverbindungen", Seiten 597-602**
**J. AM. CHEM. SOC., Band 60, Mai 1938, WASHINGTON (US), J.L. RIEBSOMER et al.: "The preparation of substituted mandelic acids and their *bacteriological effects I", Seiten 1015-1016***
**CHEMICAL ABSTRACTS, Band 59, 14. Oktober 1963, Nr. 8, Kolom 8610b, COLUMBUS, Ohio (US)**
**CHEMICAL ABSTRACTS, Band 31, 20. September 1937, Spalte 6632, COLUMBUS, Ohio (US)**
**ACTA CHEMICA SCANDINAVICA, Band 15, Nr. 2, 1961, KOPENHAGEN (DK), S.O. LAWESSON et al.: "Studies on peroxy compounds XI. The introduction of the t-butoxy- and benzoyloxy**

(73) Patentinhaber: **BAYER AG, *Konzernverwaltung* RP Patentabteilung, D-5090 Leverkusen 1 Bayerwerk (DE)**

(72) Erfinder: **Fauss, Rudolf, Dr., Gerstenkamp 10, D-5000 Koeln 80 (DE)**
Erfinder: **Lantzsch, Reinhard, Dr., Heymannstrasse 32, D-5090 Leverkusen (DE)**
Erfinder: **Findeisen, Kurt, Dr., In der Follmuehle 10, D-5068 Odenthal (DE)**
Erfinder: **Jäger, Gerhard, Dr., Gellertstrasse 18, D-5090 Leverkusen 1 (DE)**
Erfinder: **Hammann, Ingeborg, Dr., Lutherstrasse 22, D-4300 Muelheim/Ruhr (DE)**
Erfinder: **Becker, Benedikt, Dr., Metzkausenerstrasse 14, D-4020 Mettmann (DE)**
Erfinder: **Homeyer, Bernhard, Dr., Obere Strasse 28, D-5090 Leverkusen 3 (DE)**

(56) Entgegenhaltungen: (Fortsetzung)
**groups, into malonic esters - a novel method for the preparation of tartronates", Seiten 260-270**
**J. AM. CHEM. SOC., Band 71, Januar 1949, WASHINGTON (US), C.S. MARVEL et al.: "Benzoyl cyanide dimer and the addition of benzoyl cyanide to aromatic aldehydes", Seiten 34-36**
**CHEMICAL ABSTRACTS, Band 52, Nr. 14, 25. Juli 1958, Spalte 11864h, COLUMBUS, Ohio (US)**

***Die Akte enthält technische Angaben, die nach*** **dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 108 351 B1**

LIBER, STOCKHOLM 1987

**Beschreibung**

Die vorliegende Erfindung betrifft die Verwendung substituierter Malonsäurederivate, als Schädlingsbekämpfungsmittel, neue substituierte Malonsäurederivate und Verfahren zur Herstellung der neuen Verbindungen.

Es ist bereits bekannt geworden, daß Carbamate wie 5,6-Dimethyl-2-dimethylamino-4-pyrimidinyl-dimethylcarbamat oder 1-Naphthyl-N-methylcarbamat insektizide Wirksamkeit besitzen (US-PS 3 493 574, US-PS 2 903 478). Ihre Wirkung ist bei niedrigen Aufwandkonzentrationen nicht immer befriedigend.

Es wurde gefunden, daß die substituierten Malonsäurederivate der allgemeinen Formel I sich hervorragend zur Schädlingsbekämpfung eignen:

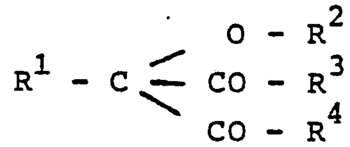 I

wobei

$R^1$ = ein- bis dreifach, gleich oder verschieden in 3,4,5-Stellung durch Halogen substituiertes Phenyl,

$R^2$ =Wasserstoff, $C_{1-4}$-Alkyl, gegebenenfalls halogensubstituiertes $C_{1-4}$-Alkyl,aminosulfonyl, $C_{1-4}$-Alkylcarbonyl, gegebenenfalls durch Halogen substituiertes Phenylcarbonyl, Phenylsulfenyl, gegebenenfalls halogensubstituiertes $C_{1-4}$-Alkylsulfenyl, Trialkylsilyl oder Rest der Formel

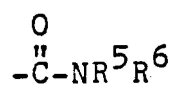

wobei $R^5$ für Wasserstoff steht und $R^6$ für gegebenenfalls durch Halogen substituiertes Phenyl oder Phenylcarbonyl oder für $C_{1-4}$-Alkyl oder $C_{1-4}$-Alkylcarbonyl steht,

$R^3$ und $R^4$ gleich oder verschieden sind und für Amino, Trimethylsilylamino, Formylamino, $C_1$-$C_4$-Alkylcarbonylamino, Alkylamino, Trialkylsilyl-alkylamino stehen, und wobei, falls $R^2$ für Wasserstoff steht, $R^3$ und $R^4$ nicht gleichzeitig für Amino stehen dürfen.

Besonders geeignet sind die erfindungsgemäß verwendbaren substituierten Malonsäurederivate zur Bekämpfung von Insekten und Spinnenmilben. Sie zeichen sich dabei durch eine erheblich bessere Wirkung aus als die aus dem Stand der Technik für diese Indikationen bekannten Verbindungen. Sie besitzen außerdem günstige Werte der Warmblütertoxizität.

Bevorzugt werden dabei die weiter unten als bevorzugt genannten neuen Verbindungen der Formel I eingesetzt.

1. Es wurden ferner die neuen substituierten Malonsäurederivate der allgemeinen Formel I gefunden

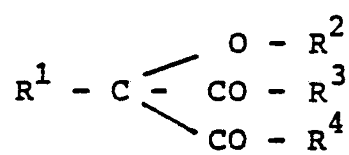 I

wobei

$R^1$ = ein- bis dreifach, gleich oder verschieden in 3,4,5-Stellung durch Halogen substituiertes Phenyl,

$R^2$ = Wasserstoff, $C_{1-4}$-Alkyl, gegebenenfalls halogensubstituiertes $C_{1-4}$-Alkyl-aminosulfonyl, $C_{1-4}$-Alkylcarbonyl, gegebenenfalls durch Halogen substituiertes Phenylcarbonyl, Phenylsulfenyl, gegebenenfalls halogensubstituiertes $C_{1-4}$-Alkylsulfenyl, Trialkylsilyl oder Rest der Formel

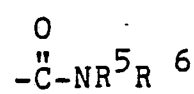

wobei $R^5$ für Wasserstoff steht und $R^6$ für gegebenenfalls durch Halogen substituierte Phenyl oder Phenylcarbonyl oder für $C_{1-4}$-Alkyl oder $C_{1-4}$-Alkylcarbonyl steht,

$R^3$ und $R^4$ gleich oder verschieden sind und für Amino, Trimethylsilylamino, Formylamino, $C_1$-$C_4$-Alkylcarbonylamino, Alkylamino, Trialkylsilyl-alkylamino stehen, und wobei, falls $R^2$ für Wasserstoff steht, $R^3$ und $R^4$ nicht gleichzeitig für Amino stehen dürfen.

Besonders seien genannt Verbindungen der Formel I in welcher

$R^1$ für Phenyl steht das
ein-oder mehrfach durch Chlor substituiert ist und

$R^2$ für Trimethylsilyl steht,

$R^3$ und $R^4$ für Trimethylsilylamino stehen.

2. Es wurde ferner gefunden, daß man die Verbindungen der Formel I

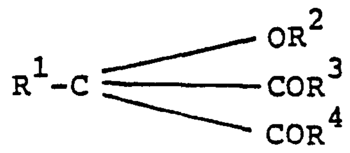

I

wobei

$R^1$ = ein- bis dreifach, gleich oder verschieden in 3,4,5-Stellung durch Halogen substituiertes Phenyl,

$R^2$ = Wasserstoff, $C_{1-4}$-Alkyl, gegebenenfalls halogensubstituiertes $C_{1-4}$-Alkylaminosulfonyl, $C_{1-4}$-Alkylcarbonyl, gegebenenfalls durch Halogen substituiertes-Phenylcarbonyl, Phenylsulfenyl, gegebenenfalls halogensubstituiertes $C_{1-4}$-Alkylsulfenyl, Trialkylsilyl oder Rest der Formel

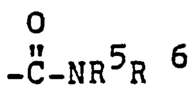

wobei $R^5$ für Wasserstoff steht und $R^6$ für gegebenenfalls durch Halogen stubstituiertes Phenyl oder Phenylcarbonyl oder für $C_{1-4}$-Alkyl oder $C_{1-4}$-Alkylcarbonyl steht,

$R^3$ und $R^4$ gleich oder verschieden sind und für Amino, Trimethylsilylamino, Formylamino, $C_1$-$C_4$-Alkylcarbonylamino, Alkylamino, Trialkylsilylalkylamino stehen, und wobei, falls $R^2$ für Wasserstoff steht, $R^3$ und $R^4$ nicht gleichzeitig für Amino stehen dürfen, erhält, indem man Verbindungen der Formel

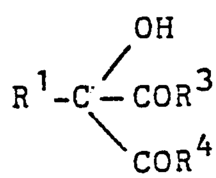

(II)

$a_1$) mit Verbindungen der Formel III

$R^{10}$-Y (III)

wobei

Y für Halogen oder $-O-SO_2-O-C_{1-4}$-Alkyl steht,

$R^{10}$ für $C_{1-4}$-Alkyl steht, umsetzt oder

$a_2$) mit Verbindungen der Formel IV

$R^{11}$-Z (IV)

wobei

Z für Halogen, $C_{1-4}$-Alkylcarbonyloxy, CN, steht,

$R^{11}$ für $C_{1-4}$-Alkylcarbonyl, gegebenenfalls durch Halogen substituiertes Phenylcarbonyl, $C_{1-4}$-Alkylaminosulfonyl steht sowie für

$-CONR^5R^6$ steht,

wobei

$R^5$ und $R^6$ die oben angegebene Bedeutung besitzen, umsetzt oder

$a_3$) mit Verbindungen der Formel V

$R^{12}$-NCO (V)

in welcher

$R^{12}$ für gegebenenfalls durch Halogen substituiertes Phenyl oder Phenylcarbonyl oder für $C_{1-4}$-Alkyl oder $C_{1-4}$-Alkylcarbonyl steht,

umsetzt oder

daß man

b) für den Fall, daß $R^2$ für Trialkylsilyl steht, Verbindungen der Formel II mit Trialkylsilyl-Verbindungen der Formel VIII

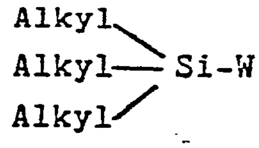

(VIII)

in welcher
W für Halogen bzw. CN, oder für Trialkylsilylamino steht,
in annähernd äquimolaren Mengen gegebenenfalls in Gegenwart eines Verdünnungsmittels, gegebenenfalls eines Katalysators und gegebenenfalls in Gegenwart eines Säureakzeptors umsetzt.

Ferner wurde gefunden:

c) Verfahren zur Herstellung von Verbindungen der Formel

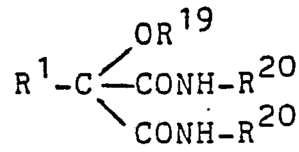

in welcher
$R^1$ die in unter Formel (I) angegebene Bedeutung besitzt,
$R^{19}$ für $C_{1-4}$-Alkylcarbonyl oder für gegebenenfalls durch Halogen substituiertes Phenylcarbonyl steht,
$R^{20}$ für Formyl oder $C_{1-4}$-Alkylcarbonyl steht, welches dadurch gekennzeichnet ist, daß man Verbindungen der Formel

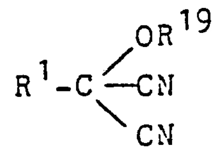

in welcher $R^1$ und $R^{19}$ die oben angegebene Bedeutung haben, mit Säuren der Formel
$R^{20}$-OH
in welcher
$R^{20}$ die oben angegebene Bedeutung hat
oder ihren Anhydriden in Gegenwart anorganischer Mineralsäuren gegebenenfalls unter Zusatz von Wasser umsetzt.

Weiterhin wurde gefunden:

d) Verfahren zur Herstellung von Verbindungen der Formel

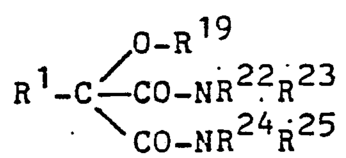

in welcher
$R^1$, $R^{19}$ die oben angegebene Bedeutung besitzen,
$R^{22}$ für $C_{1-4}$-Alkylcarbonyl
$R^{24}$ für Wasserstoff oder $C_{1-4}$-Alkylcarbonyl steht,
$R^{23}$ für Wasserstoff oder Alkyl steht,
$R^{25}$ für Wasserstoff oder Alkyl steht,
welches dadurch gekennzeichnet ist, daß man Verbindungen der Formel

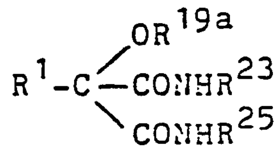

wobei $R^1$, $R^{23}$, $R^{25}$ die oben angegebene Bedeutung besitzen,
$R^{19a}$ für Wasserstoff, $C_{1-4}$-Alkylcarbonyl oder für gegebenenfalls durch Halogen substituiertes Phenylcarbonyl steht,
mit Säureanhydriden der allgemeinen Formel

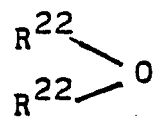

in welcher
$R^{22}$ die oben angegebene Bedeutung hat, umsetzt.
Weiterhin wurde gefunden:
e) Verfahren zur Herstellung von Verbindungen der Formel

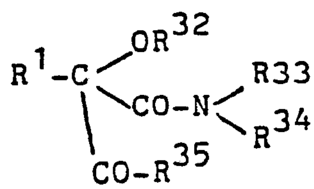

in welcher
$R^1$ die oben angegebene Bedeutung hat,
$R^{32}$ für Trialkylsilyl, $C_{1-4}$-Alkyl, $C_{1-4}$-Alkylcarbonyl, gegebenenfalls durch Halogen substituiertes Phenylcarbonyl steht,
$R^{33}$ für Wasserstoff steht,
$R^{34}$ für Trimethylsilyl steht,
$R^{35}$ für Trimethylsilylamino, Trialkylsilylalkylamino, Alkylamino steht welches
dadurch gekennzeichnet ist, daß man Verbindungen der Formel

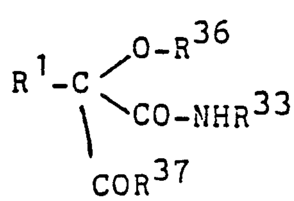

in welcher
$R^1$ und $R^{33}$ die oben angegebene Bedeutung haben und
$R^{36}$ für Wasserstoff, $C_{1-4}$-Alkyl, $C_{1-4}$-Alkylcarbonyl, gegebenenfalls durch Halogen substituiertes Phenylcarbonyl steht,
$R^{37}$ für Amino, Alkylamino steht,
mit Trialkylsilylverbindungen der Formel

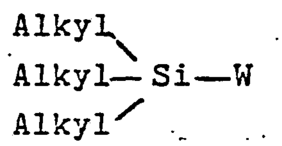

in welcher W die oben angegebene Bedeutung besitzt, umsetzt.

Ferner wurde gefunden:

f) Verfahren zur Herstellung von Verbindungen der Formel XXIV

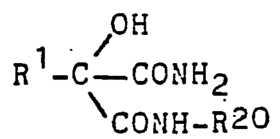

(XXIV)

in welcher

$R^1$ die in Anspruch 1 angegebene Bedeutung hat und

$R^{20}$ für Formyl, Alkylcarbonyl oder gegebenenfalls durch Halogen substituiertes Phenylcarbonyl steht, welches

dadurch gekennzeichnet ist, daß man Verbindungen der Formel XXV

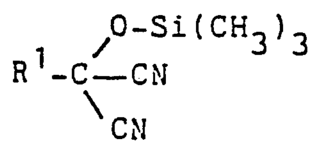

(XXV)

in welcher

$R^1$ die oben angegebene Bedeutung hat, mit Säuren der Formel XXIII

$R^{20}$-OH (XXIII)

in welcher

$R^{20}$ die oben angegebene Bedeutung hat

oder ihren Anhydriden in Gegenwart von anorganischen Mineralsäuren umsetzt.

Im folgenden wird die Durchführung der unteren erwähnten Verfahren näher beschrieben:

Verfahren $2a_1$

Die Umsetzung der Verbindungen der Formeln II und III erfolgt gegebenenfalls in Gegenwart von Säureakzeptoren, gegebenenfalls in Gegenwart eines Katalysators, gegebenenfalls in Gegenwart eines Verdünnungsmittels. Der Reaktionsablauf kann z. B. durch das folgende Formelschema wiedergegeben werden:

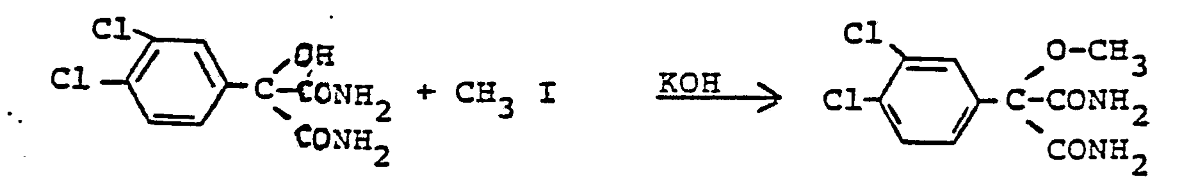


Die Verbindungen der Formeln II und III werden in äquimolaren Mengen eingesetzt, ein Überschuß der einen oder anderen Komponente bringt keine wesentlichen Vorteile.

Als Verbindungen der Formeln II werden bevorzugt diejenigen eingesetzt die in den Suhstituenten $R^3$, $R^4$ die bei den Verbindungen der Formel I genannten bevorzugten Bedeutungen besitzen. Die Verbindungen der Formel II in welcher $R^2$ für Wasserstoff und $R^3$ und $R^4$ gleichzeitig für Amino stehen sind Gegenstand der älteren, am 20.04.83 veröffentlichten europäischen Anmeldung 007 69 57. Sie lassen sich nach den dort angegebenen Verfahren herstellen. Sie lassen sich auch nach den unten beschriebenen Verfahren herstellen. Im einzelnen seien die folgenden Verbindungen der Formel II genannt:

2-, 3-, 4-Chlorphenylhydroxy-malonsäurediamid, 2,3-Di-chlorphenyl-hydroxymalonsäurediamid, 3,4-Dichlorphenyl-hydroxy-malonsäurediamid, 3,5-Dichlorphenyl-hydroxy-malonsäurediamid, 2,4-Dichlorphenyl-hydroxy-malonsäurediamid, 2,5-Dichlorphenyl-hydroxy-malonsäurediamid, 2,6-Dichlorophenyl-hydroxy-

malonsäurediamid, 3-,3-, und 4-Fluorphenyl-hydroxymalonsäurediamid, 3,4-Dichlorphenyl-hydroxymalonsäureamid-methylamid, 2,3,4-; 2,3,6-; 3,4,5-Trichlorphenyl-hydroxy-malonsäurediamid.

Als Beispiele für die Ausgangsstoffe der Formel (III) seien genannt:

Methylbromid, Methyliodid, Ethylbromid, Ethyliodid, 1-Iodpropan, 2-Iodpropan.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethyleter, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Dimethylsulfoxid, Tetramethylensulfon und Hexamethylphosphorsäuretriamid.

Als Säureakzeptoren können alle üblichen Säurebindemittel verwendet werden. Hierzu gehören vorzugsweise Alkalicarbonate, -hydroxide oder -alkoholate, wie Natrium- oder Kaliumcarbonat, Natrium- und Kaliumhydroxid, Natrium- und Kaliummethylat bzw. -ethylat, ferner aliphatische, aromatische oder heterocyclische Amine, beispielsweise Trimethylamin, Triethylamin, Tributylamin, Dimethylanilin, Dimethylbenzylamin und Pyridin.

Als Katalysatoren können Verbindungen verwendet werden, welche gewöhnlich bei Reaktionen in Zweiphasensystemen aus Wasser und mit Wasser nicht mischbaren organischen Lösungsmitteln zum Phasentransfer von Reaktanden dienen (Phasentransferkatalysatoren). Als solche sind vor allem Tetraalkyl- und Trialkylaralkyl-ammoniumsalze mit vorzugsweise 1 bis 10, insbesondere 1 bis 8 Kohlenstoffen je Alkylgruppe, vorzugsweise Phenyl als Arylbestandteil der Aralkylgruppe und vorzugsweise 1 bis 4, insbesondere 1 oder 2 Kohlenstoffatomen im Alkylteil der Aralkylgruppen bevorzugt. Hierbei kommen vor allem die Halogenide, wie Chloride, Bromide und Iodide, vorzugsweise die Chloride und Bromide infrage. Beispielhaft seien Tetrabutylammoniumbromid, Benzyl-triethylammoniumchlorid und Methyltrioctylammoniumchlorid genannt.

Die Reaktionstemperatur wird zwischen etwa 0°C und 130°C, vorzugsweise zwischen etwa 20°C und 60°C gehalten. Das Verfahren wird vorzugsweise bei Normaldruck durchgeführt.

Die Isolierung der Endprodukte erfolgt in allgemein üblicher Weise.

Verfahren $2a_2$

Die Umsetzung der Verbindungen der Formeln II und IV erfolgt gegebenenfalls in Gegenwart von Säureakzeptoren, gegebenenfalls in Gegenwart eines Katalysators, gegebenenfalls in Gegenwart eines Verdünnungsmittels.

Der Reaktionsablauf kann z. B. durch das folgende Formelschema wiedergegeben werden:

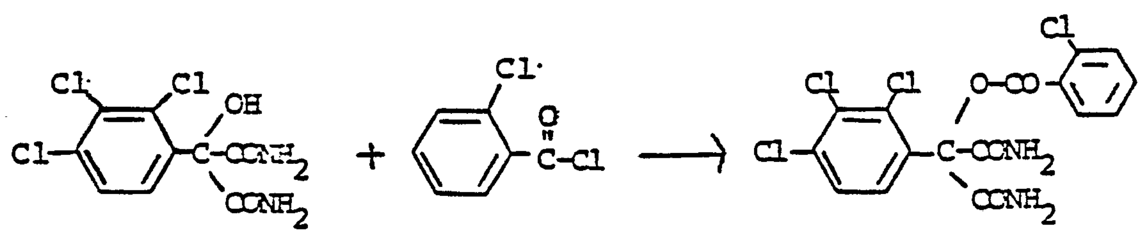

Bevorzugt seien auch hier die bei Verfahren $2a_1$ aufgeführten Verbindungen der Formel II genannt.

Als Verbindungen der Formel IV seien bevorzugt genannt:

Benzoylchlorid, 3,4-Dichlorbenzoylchlorid, 3-Chlorbenzoylbromid, Acetylchlorid, Acetanhydrid, Propionylchlorid, Benzoylcyanid, Chlorameisensäureethylester, Chlorameisensäuremethylester.

Das Verfahren kann entweder in Gegenwart eines Säureakzeptors oder ohne Base durchgeführt werden. Wird in Gegenwart eines Säureakzeptors gearbeitet, werden die Verbindungen der Formeln II und IV in äquimolaren Verhältnissen eingesetzt, ein geringer Überschuß der einen oder anderen Komponente bringt keine wesentlichen Vorteile.

Als Verdünnungsmittel kommen die bei Verfahren $2a_1$ genannten infrage.

Als Säureakzeptoren kommen die bei Verfahren $2a_1$ genannten infrage. Diese Säureakzeptoren wirken zum Teil auch als Katalysatoren. Weitere Katalysatoren sind beim Verfahren $2a_1$ genannt.

Der Säureakzeptor wird bezogen auf die Verbindung IV vorzugsweise mindestens äquimolar eingesetzt. Er kann auch, im Überschuß eingesetzt, als Verdünnungsmittel dienen.

Einige Säureakzeptoren, bevorzugt die tert. Amine z. B. Triethylamin, Pyridin, können in geringer Menge als Katalysatoren zugesetzt werden und beeinflussen die Reaktion günstig.

Das Verfahren wird zwischen 0 und 130°C bevorzugt zwischen 10 und 60°C durchgeführt. Man arbeitet vorzugsweise bei Normaldruck. Die Isolierung der Endprodukte erfolgt in allgemein üblicher Weise.

Nach der bisher beschriebenen Arbeitsweise für Verfahren $2a_2$ wird eine Umsetzung der Verbindungen der Formel II nur an der Hydroxylgruppe erreicht.

Eine weitere Arbeitsweise für Verfahren $2a_2$ besteht darin, daß die Verbindung der Formeln II und IV ohne Gegenwart von Säureakzeptoren gegebenenfalls in Gegenwart von Verdünnungsmitteln und oder Katalysatoren erhitzt werden.

In diesem Fall werden die Verbindungen der Formel IV in mindestens äquimolarer Menge, bevorzugt in überschuß (1,1 - 10 fach bevorzugt 1,2 - 5 fach insbesondere 1,3- 3 fach) ein gesetzt.

Die Verdünnungsmittel sind dieselben wie unter $2a_1$ beschrieben.

Die Reaktion wird zwischen 50 und 200°C bevorzugt zwischen 70 und 150°C durchgeführt. Man arbeitet vorzugsweise bei Normaldruck.

Als Katalysatoren kommen die bei Verfahren $2a_1$ genannten infrage. Die bei dieser Arbeitsweise freigesetzten Verbindungen H-Z (z. B. HCl, HCN) werden während der Reaktion entfernt.

Nach der bisher beschriebenen Arbeitsweise wird eine Umsetzung der Verbindungen der Formel II an der Hydroxylgruppe erreicht.

Werden als Verbindungen der Formel IV Säureanhydride eingesetzt, erfolgt normalerweise eine Umsetzung der Verbindungen der Formel IV sowohl an der Hydroxylgruppe als auch, falls möglich, an der Amidgruppe.

Unter bestimmten Bedingungen kann jedoch auch die Reaktion mit den Säureanhydriden auf die Umsetzung der Hydroxylgruppe der Verbindungen der Formel II beschränkt bleiben.

Dazu ist erforderlich, daß Verbindung II und Säureanhydrid nur in etwa äquimolarem Verhältnis eingesetzt werden.

Allgemein gilt, daß bei der Umsetzung mit Säureanhydriden wie oben beschrieben gearbeitet wird. Als Katalysatoren finden jedoch außerdem Säuren wie Mineralsäuren z. B. HCl, $H_2SO_4$, $H_3PO_4$, $HClO_4$ oder starke organische Säuren z. B. p-Toluolsulfonsäure oder Trifluormethansulfonsäure, Verwendung. Ebenso können Lewis-Säuren wie z. B. $AlCl_3$, $BF_3$, ZnCl, $FeCl_3$ hierfür eingesetzt werden.

Verfahren $2a_3$

Die Umsetzung der Verbindungen der Formel II und V erfolgt gegebenenfalls in Gegenwart eines Katalysators, gegebenenfalls in Gegenwart eines Verdünnungsmittels.

Der Reaktionsablauf läßt sich durch das folgende Formelschema wiedergeben.

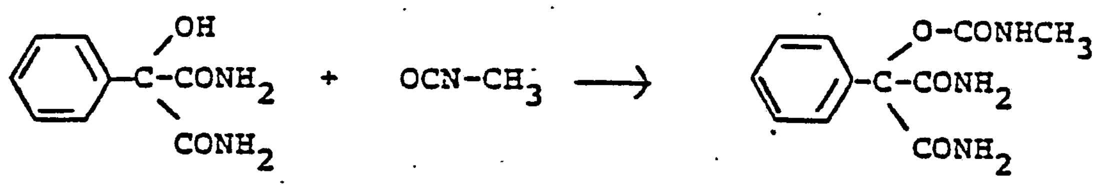

Bevorzugt seien auch hier die bei Verfahren $2a_1$ aufgeführten Verbindungen der Formel II genannt.

Als Verbindungen der Formel V seien bevorzugt genannt:

Isocyanate, die sich von den folgenden Aminen ableiten:

Methylamin, Ethylamin, Propylamin, Isopropylamin, Butylamin, iso-Butylamin, tert.-Butylamin, Anilin, o,m,p-Chlor-anilin, 2,3-, 2,4-, 2,5-, 2,6-Dichloranilin, 3,4-, 3,5-Dichloranilin.

Ferner Isocyanate wie z. B. 3,4-, 3,5-Dichlor-benzoylisocyanat, 4-Chlor-benzoylisocyanat, 3-Chlor-benzoylisocyanat, 2,6-Dichlor-benzoylisocyanat, Isopropionylisocyanat.

Diese Isocyanate sind bekannte Verbindungen. Sie lassen sich nach an sich bekannten Verfahren herstellen.

Als Verdünnungsmittel kommen die bei Verfahren $2a_1$ genannten infrage.

Als Katalysatoren kommen die bei Umsetzungen mit Isocyanaten üblichen Katalysatoren infrage. Als solche seien genannt; tert. Amine wie Triethylamin, N-Methylmorpholin, 1,4-Diaza-bicyclo-(2,2,2)-octan (DABCO) β,β'-Dimethylaminodiethylether, Dimethylbenzylamin, Metallkatalysatoren des Zn; Sn, Pb wie Dibutylzinndilaurat, Dibutylzinnoxid, Zinnoctoat, Bleioctoat, Zinkoctoat, Zinkchlorid, Zinkacetat.

Die Reaktion wird zwischen 50 und 150°C bevorzugt zwischen 60 - 110°C durchgeführt. Man arbeitet vorzugsweise unter Normaldruck.

Die Verbindungen der Formeln II und V werden in äquimolaren Mengen eingesetzt ein geringer überschuß der einen oder anderen Komponente bringt keine wesentlichen Vorteile.

Die Endprodukte werden im allgemeinen üblicher Weise isoliert.

Verwendet man beispielsweise für Verfahren 2b als Ausgangsstoffe Phenylhydroxymalonsäurediamid und Trimethylsilylcyanid, so kann die Reaktion durch folgendes Formelschema skizziert werden:

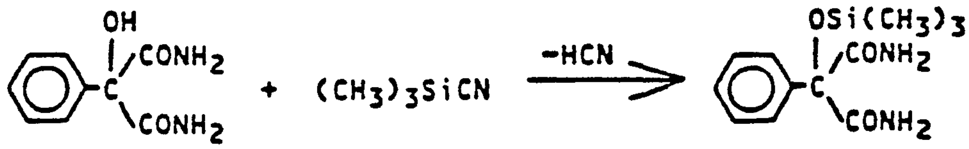

Die als Ausgangsstoffe zu verwendenden Hydroxy-malonsäureverbindungen sind durch die Formel (II) definiert. Bevorzugt seien hier die bei Verfahren $2a_1$ aufgeführten Verbindungen der Formel II genannt.

Die Trialkylsilylderivate der Formel VIII sind bekannt. Bevorzugt steht in der allgemeinen Formel Alkyl für gleiche oder verschiedene $C_{1-4}$-Alkylreste wie Methyl, Ethyl. Bevorzugt steht der Substituent W für die Reste Halogen wie Chlor, sowie für CN.

Im einzelnen seien genannt:

Trimethylsilylchlorid, Trimethylsilylbromid, Trimethylsilyliodid, Trimethylsilylcyanid, tert.-Butyldimethylsilylchlorid bzw. -cyanid.

Als Verdünnungsmittel kommen die bei Verfahren $2a_1$ genannten infrage.

Als Säureakzeptoren und Katalysatoren kommen die bei Verfahren $2a_1$ genannten infrage.

Die Reaktionstemperatur liegt zwischen etwa 0°C und 130°C, vorzugsweise zwischen etwa 20°C und 110°C. Das Verfahren wird vorzugsweise bei Normaldruck durchgeführt.

Zur Durchführung des erfindungsgemäßen Verfahrens (2b) werden die Verbindungen der Formel (II) und die Trialkylsilylverbindungen gewöhnlich in äquivalenten Mengen eingesetzt.

Die Isolierung der Endprodukte erfolgt in allgemein üblicher Weise.

Verfahren c)

Die Umsetzung der Verbindungen der Formel

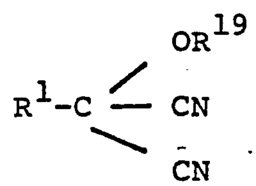

mit Säuren der Formel $R^{20}$-OH erfolgt gegebenfalls in Gegenwart von Verdünnungsmitteln, gegebenenfalls in Gegenwart von Katalysatoren.

Verfahren d)

Die Umsetzung der Verbindungen der Formel

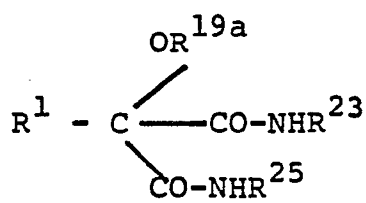

mit den Acylierungsmitteln der Formel

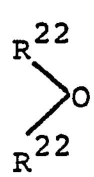

erfolgt gegebenenfalls in Gegenwart von Verdünnungsmitteln und gegebenenfalls in Gegenwart von Katalysatoren.

Verbindungen der Formel

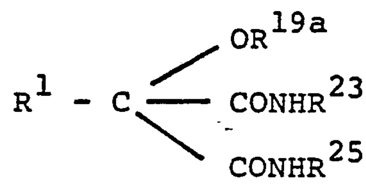

sind zum Teil neu, zum Teil Gegenstand der europäischen Anmeldung 007 69 57.

Verbindungen der Formel

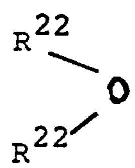

sind bekannt. Bevorzugt wird Acetanhydrid eingesetzt.

Als Verdünnungsmittel kommen die bei Verfahren $2a_1$ genannten infrage.

Als Säureakzeptoren kommen die bei Verfahren $2a_1$ genannten infrage.

Die Verbindungen der Formeln

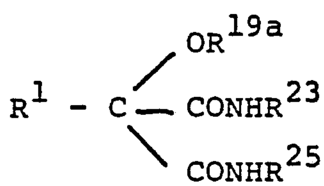

und

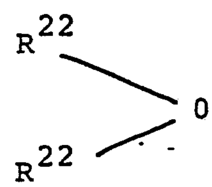

werden bevorzugt im stöchiometrischen Verhältnis (1:1) umgesetzt.

Die Aufarbeitung erfolgt in üblicher Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp..

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis

chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp..

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische-Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte

Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variiert werden. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten vorzugsweise von ektoparasitierenden Insekten auf dem Gebiet der Tierhaltung und der Tierzucht.

Die Anwendung der erfindungsgemäßen Wirktoffe geschieht hier in bekannter Weise, wie durch orale Anwendung, durch dermale Anwendung in Form beispielsweise des Tauchens (Dippen), Sprühens (Sprayen), Aufgießens (pur-on and spot-on) und des Einpuderns.

## Herstellungsbeispiele

### Beispiel 1

Verfahren $2a_1$

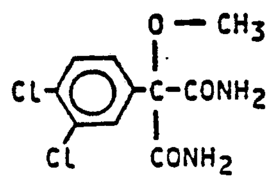

30 g (0,114 Mol) 3,4-Dichlor-phenyl-hydroxy-malonsäurediamid werden in 60 ml Dimethylsulfoxid vorgelegt. Dann werden 6,38 g (0,144 Mol) Kaliumhydroxid in 150 ml Wasser, anschließend 16,1 g (0,114 Mol) Methyliodid zugetropft. Die Reaktion ist exotherm: Die Temperatur steigt auf etwa 50°C. Man läßt abkühlen und rührt 16 Stunden bei 20°C nach. Der ausgefallene Feststoff wird abgesaugt und mit Wasser und dann mit Petrolether gewaschen.

Nach Umkristallisieren aus Essigester erhält man 14,1 g (44,3 % der Theorie) 2-(3,4-Dichlorphenyl)-2-methoxy-malonsäurediamid vom Schmelzpunkt 211 bis 213°C.

### Beispiel 2

Verfahren $2a_2$

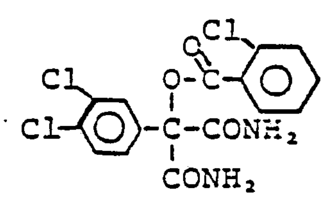

26,3 g 3,4-Dichlorphenylhydroxymalonsäurediamid wurde in 50 ml Pyridin gelöst und bei unter 30°C mit 17,1 g o-Chlorbenzoylchlorid versetzt. Nach einer Stunde wurde in kalte 1n HCl eingerührt, der Niederschlag in Essigsäureethylester aufgenommen, ausgeschüttelt und aufgearbeitet. Es blieben 26,5 g Rohprodukt, welches auf 400 ml Ethanol umkristallisiert wurde. Es wurden 13 g reines 3,4-Dichlorphenyl-(2-chlorbenzoyloxy)-malonsäurediamid vom Fp. 220°C erhalten.

**Beispiel 2a**

Analog obigem Verfahren wurde 3,4-Dichlorphenyl-(3,4-dichlorbenzoyloxy)-malonsäurediamid vom Fp. 237°C (Zersetzung) erhalten.

**Beispiel 3**

Verfahren $2a_2$

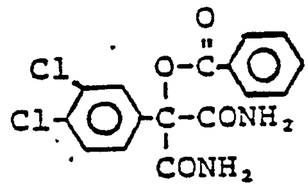

13,1 g 3,4-Dichlorphenylhydroxymalonsäurediamid wurden in 200 ml Benzoylchlorid 9 Stunden auf 110°C erhitzt. Anschließend wurde auf 5°C abgekühlt, abgesaugt, der Niederschlag mit Toluol gewaschen und anschließend aus 550 ml i-Propanol umkristallisiert. Es wurden 10 g 3,4-Dichlorphenylbenzoyloxymalonsäurediamid isoliert: Fp. 225°C unter Zersetzung.

**Beispiel 4**

Verfahren $2a_2$

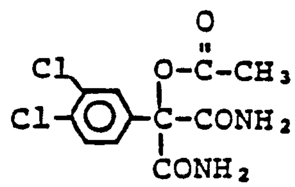

131 g 3,4-Dichlorphenylhydroxymalonsäurediamid, 850 ml Tetrahydrofuran und 750 ml Acetylchlorid wurden 4 Stunden am Rückfluß erhitzt. Nach Abkühlen wurder der Niederschlag abgesaugt und mit i-Propanol gewaschen. Es wurden 115 g 3,4-Dichlorphenylacetoxymalonsäurediamid vom Fp. = 206°C erhalten.

**Beispiel 5**

Verfahren $2a_2$

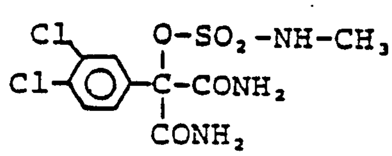

26,3 g 3,4-Dichlorphenylhydroxymalonsäurediamid wurden in 70 ml Pyridin gelöst und bei Raumtemperatur mit 18 g Methylsulfamoylchlorid versetzt.

Nach 3 Stunden wurde in kalte 1n HCl eingerührt, mit Essigsäureethylester extrahiert und die organische Phase aufgearbeitet. Es wurden 17 g 3,4-Dichlorphenyl-methylsulfamoyloxymalonsäurediamid vom Fp. = 124°C isoliert.

Verfahren $2a_3$

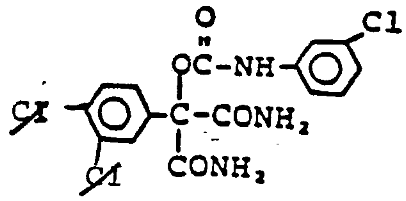

8,2 Phenyl-hydroxy-malonsäure-diamid wurde in 30 ml Essigsäureethylester mit 0,5 g Zinkoctoat und 4,45 g 3-Chlorphenylisocyanat versetzt und 30 min. auf 50° C gehalten. Nach Abkühlen wurde abgesaugt und aus Aceton umkristallisiert.

**Beispiele**

Nach obiger Vorschrift wurden folgende Isocyanat-umsetzungsprodukte erhalten:

(6)

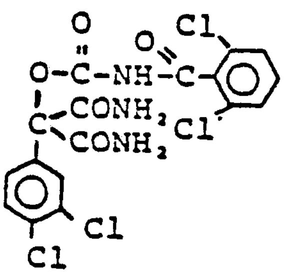

Fp.: 189 - 191°C

7)

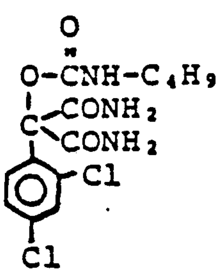

Fp.= 189 - 191°C

8)

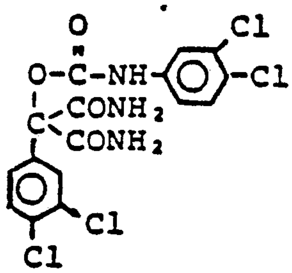

Fp.= 193 - 194°C

9)

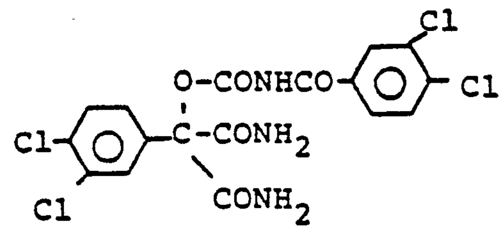

Fp. = 199°C

**Beispiel 10**

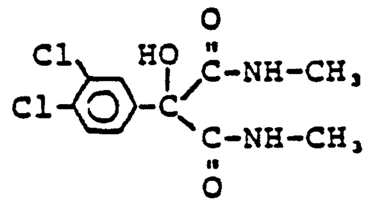

14,7 g 3,4-Dichlorphenylhydroxymalonsäuredimethylester wurden in 100 ml Methanol gelöst undbei 60°C 1 Stunde Methylamin eingeleitet. Es wurde völlig eingeengt und der kristalline Rückstand aus 100 ml Ethanol umkristallisiert. Es wurden 12 g 3,4-Dichlorphenyl-hydroxymalonsäuredimethylamid (Fp. = 158 - 160°C) erhalten.

**Beispiel 11**

Analog zu obigem Verfahren wurde 3,4-Dichlorphenyl-hydroxymalonsäuredineopentylamid (Fp. = 94 - 95°C) erhalten.

**Beispiel 12**

Verfahren $2a_2$

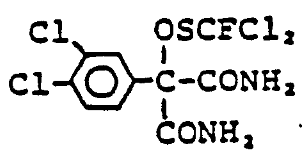

13 g 3,4-Dichlorphenylhydroxymalonsäurediamid und 8,5 g Dichlorfluormethylsulfonylchlorid wurden in 100 ml Dioxan 10 Stunden am Rückfluß erhitzt. Nach dem Einengen blieben 20 g 3,4-Dichlorphenyl-dichlorfluormethylsulfenyloxy-malonsäurediamid vom Fp. 120°C (Zersetzung).

**Beispiel 13**

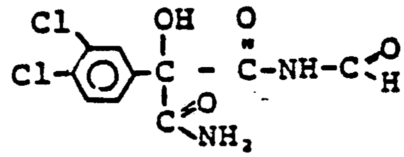

In 67 g Ameisensäure und 16,8 ml $H_2SO_4$ konz. wurden 77,3 g 3,4-Dichlorphenyltrimethylsilyloxymalonsäuredinitril bei 25° C zugetropft und 28 Stunden bei dieser Temperatur unter gelegentlichem Rühren gehalten. Anschließend wurde in Eiswasser eingerührt, mit Essigsäureethylester aufgenommen und die organische Phase neutral gewaschen. Nach dem Aufarbeiten blieben 48 g Rohprodukt, das aus Isopropanol umkristallisiert wurde. Nach spektroskopischen Untersuchungen (IR, $H^1$- und $C^{13}$-NMR, MS (DCI) lag 3,4-Dichlorphenylhydroxymalonsäureamidformylamid neben 3,4-Dichlorphenyl-hydroxymalonsäurediamid im ungefähren Verhältnis 1:1 vor.

**Beispiel 14**

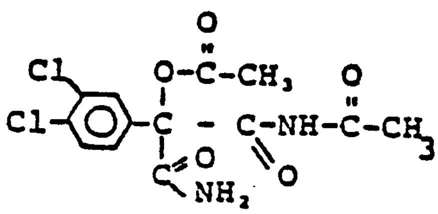

13,2 g 3,4-Dichlorphenylhydroxymalonsäurediamid wurden in 60 ml Acetanhydrid auf 80° C erhitzt und bei dieser Temperatur 6 ml einer Lösung aus 0,3 g $H_2SO_4$ konz. in 100 ml Acetanhydrid zugegeben. Es wurde 10 min. bei 80° C weitergerührt, dänn auf 20° C abgekühlt, auf Eis gegossen und das Rohprodukt abgesaugt. Nach dem Trocknen wurde in Methylenchlorid digeriert und abgesaugt. Es blieben 8 g 3,4-Dichlorphenyl-acetoxymalonsäureamidacetylemid vom Fp. 202° C (Zersetzung).

**Beispiel 15**

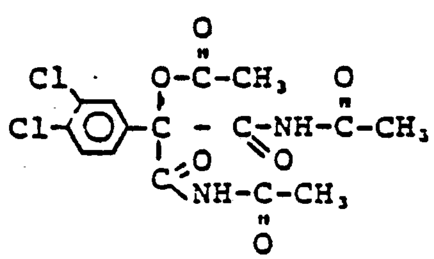

52,6 g 3,4-Dichlorphenylhydroxymalonsäurediamid wurden in 130 ml Acetanhydrid suspendiert und mit 0,3 ml $H_2SO_4$ konz. versetzt. Es wurde 45 min auf 80° erwärmt, wobei eine klare Lösung entstand. Die nach dem Abkühlen ausgefallenen Kristalle wurden scharf abgesaugt, mit Wasser und wenig i-Propanol digeriert. Es blieben 55 g 3,4-Dichlorphenyl-acetoxy-malonsäurebisacetyl-amid vom Fp. 170° C.

**Beispiel 16**

Analog obigem Beispiel wurden 12 g 4-Chlor-3-methyl-phenylhydroxymalonsäurediamid umgesetzt. Es wurden 14,3 g 4-Chlor-3-methylphenyl-acetoxymalonsäurebis-acetylamid vom Fp. 169 - 171° C erhalten.

**Beispiel 17**

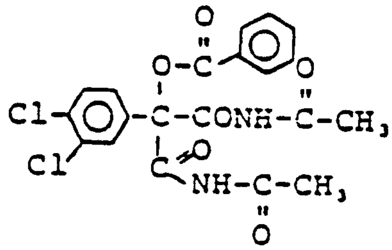

7,7 g 3,4-Dichlorphenylbenzoyloxymalonsäurediamid wurden in 60 ml Acetanhydrid mit 3 Tropfen $H_2SO_4$ konz. versetzt und 7 Stunden auf 75° C erhitzt. Anschließend wurde auf Eiswasser gegossen, abgesaugt und aus i-Propanol umkristallisiert. Es wurden 4 g 3,4-Dichlorphenyl-benzoyloxymalonsäurebisacetylamid erhalten Fp. 198° C (Zersetzung).

**Beispiel 18**

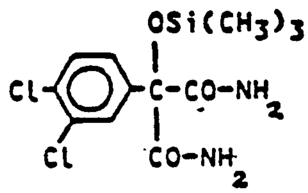

3 g (0,0114 Mol) 3,4-Dichlor-phenyl-hydroxy-malonsäurediamid, 1,12 g (0,114 Mol) Trimethylsilylcyanid und 0,9 g (0,0114 Mol) Pyridin werden 6 Stunden auf 100° C (Badtemperatur) erhitzt. Nach Entfernen des Pyridins im Wasserstrahlvakuum wird der Rückstand aus Petrolether umkristallisiert.

Man erhält 3,45 g (90,3 g der Theorie) 2-(3,4-Dichlorphenyl)-2-trimethylsilyloxy-malonsäurediamid vom Schmelzpunkt 156° C.

**Beispiel 19**

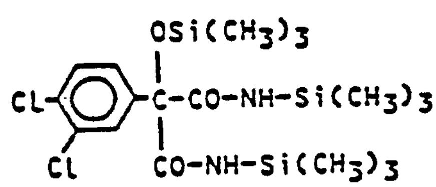

30 g (0,114 Mol) 3,4-Dichlor-phenyl-hydroxy-malonsäurediamid, 46 g (0,464 Mol) Trimethylsilylcyanid und einige Tropfen Pyridin werden gemischt und 12 Stunden zum Sieden erhitzt. Nach dem Abkühlen saugt man ab und wäscht mit wenig kaltem Petrolether.

Man erhält 48,6 g (89 % der Theorie) 2-(3,4-Dichlorphenyl)-2-trimethylsilyl-1,3-bis-trimethylsilylmalonsäurediamid vom Schmelzpunkt 107 bis 109° C.

Es wurde vorgegangen wie in obigem Beispiel, jedoch ohne Pyridin als Katalysator, dafür in 130 ml Dioxan als Lösungsmittel. Nach Einengen und Umkristallisation aus Waschbenzin wurde auch hier das gewünschte Endprodukt erhalten.

Analog Beispiel 19 können die folgenden Verbindungen der Formel (I) hergestellt werden:

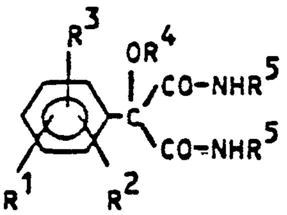

(I)

| Beispiel Nr. | $R^1$ | $R^2$ | $R^3$ | $R^4$ | $R^5$ | Schmelzpunkt ° |
|---|---|---|---|---|---|---|
| 20 | 4-Cl | H | H | $-Si(CH_3)_3$ | $-Si(CH_3)_3$ | 134 |
| 21 | 3-Cl | 5-Cl | H | $-Si(CH_3)_3$ | $-Si(CH_3)_3$ | 112-113 |
| 22 | 3-Cl | 3-Cl | 5-Cl | $-Si(CH_3)_3$ | $-Si(CH_3)_3$ | 236 |

**Beispiel 23**

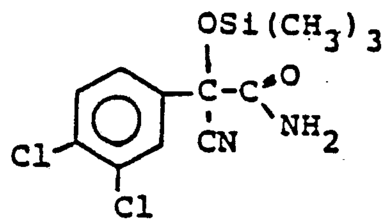

32,7 g 3,4-Dichlorphenylglyoxylsäureamid wurden in 100 ml Methylenchlorid suspendiert, mit 0,2 ml Triethylamin versetzt und 15 g Trimethylsilylcyanid zugetropft. Es wurde 1 Stunde bei Raumtemperatur nachgerührt, eingeengt und aus Toluol umkristallisiert. Es wurden 41 g 3,4-Dichlorphenyltrimethylsilyloxymalonsäureamidnitril erhalten. Fp. 148 - 149° C.

**Beispiel 24**

5 g des Produktes wurden in 25 ml konzentrierter Schwefelsäure 1 Stunde bei 25° C gerührt; die Lösung auf Eis gegeben, abgesaugt und neutral gewaschen. Es fielen 3,2 g 3,4-Dichlorphenylhydroxymalonsäurediamid an.

**Beispiel A**

Plutella-Test
Lösungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z. B. die folgenden Verbindungen der Herstellungsbeispiele überlegene Wirksamkeit gegenüber dem Stand der Technik: 13, 4, 14, 15, 5, 12, 3, 2, 17, 8, 6, 9, 1, 18, 21, 19.

**Beispiel B**

Tetranychus-Test (resistent)
Lösungsmittel: 3 Gewichtsteile Dimethylformamid
Emulgator: 1-Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 13, 14, 15, 5, 12, 17, 8, 6, 9, 1, 18, 20, 21, 19.

**Beispiel C**

Test mit Lucilia cuprina res.-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigte z. B. die Verbindung des Beispiels (18) bei einer Wirkstoffkonzentration von 1000 ppm eine 100 %-ige Abtötung.

**Beispiel D**

Grenzkonzentrations-Test / Wurzelsystemische Wirkung
Testinsekt: Myzus pesicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1,18.

**Beispiel E**

Grenzkonzentrations-Test / Wurzelsystemische Wirkung
Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat

mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 20.

**Beispiel F**

Grenzkonzentrations-Test / Bodeninsekten
Testinsekt: Phorbia antiqua (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1, 18, 19, 15, 14.

**Patentansprüche**

1. Schädlingsbekämpfungsmittel, gekennzeichnet durch einen Gehalt en substituierten Malonsäurederivaten der allgemeinen Formel I

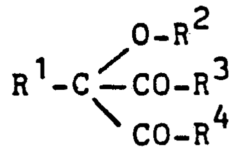

(I)

wobei

$R^1$ = ein- bis dreifach, gleich oder verschieden in 3,4,5-Stellung durch Halogen substituiertes Phenyl

$R^2$ = Wasserstoff, $C_{1-4}$-Alkyl, gegebenenfalls halogensubstituiertes $C_{1-}$-Alkylaminosulfonyl, $C_{1-4}$-Alkylcarbonyl, gegebenenfalls durch Halogen substituiertes Phenylcarbonyl, Phenylsulfenyl, gegebenenfalls halogensubstituiertes $C_{1-4}$-Alkylsulfenyl, Trialkylsilyl oder Rest der Formel

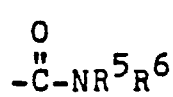

wobei $R^5$ für Wasserstoff steht und $R^6$ für gegebenenfalls durch Halogen substituiertes Phenyl oder Phenylcarbonyl oder für $C_{1-4}$-Alkyl oder $C_{1-4}$-Alkylcarbonyl steht,

$R^3$ und $R^4$ gleich oder verschieden sind und für Amino, Trimethylsilylamino, Formylamino, $C_1$-$C_4$-Alkylcarbonylamino, Alkylamino, Trialkylsilyl-alkylamino stehen, und wobei, falls $R^2$ für Wasserstoff steht, $R^3$ und $R^4$ nicht gleichzeitig für Amino stehen dürfen.

2. Substituierte Malonsäurederivate der allgemeinen Formel

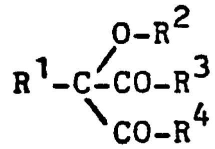

(I)

wobei

$R^1$ = ein- bis dreifach, gleich oder verschieden in 3,4,5-Stellung durch Halogen substituiertes Phenyl

$R^2$ = Wasserstoff, $C_{1\text{-}4}$-Alkyl, gegebenenfalls halogensubstituiertes $C_{1\text{-}4}$-Alkyl-aminosulfonyl, $C_{1\text{-}4}$-Alkylcarbonyl, gegebenenfalls durch Halogen substituiertes Phenylcarbonyl, Phenylsulfenyl, gegebenenfalls halogensubstituiertes $C_{1\text{-}4}$-Alkylsulfenyl, Trialkylsilyl oder Rest der Formel

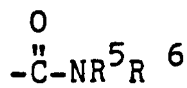

wobei $R^5$ für Wasserstoff steht und $R^6$ für gegebenenfalls durch Halogen substituierte Phenyl oder Phenylcarbonyl oder für $C_{1\text{-}4}$-Alkyl oder $C_{1\text{-}4}$-Alkylcarbonyl steht,

$R^3$ und $R^4$ oder verschieden sind und für Amino, Trimethylsilylamino, Formylamino, $C_1$-$C_4$-Alkylcarbonylamino, Alkylamino, Trialkylsilyl-alkylamino stehen, und wobei, falls $R^2$ für Wasserstoff steht, $R^3$ und $R^4$ nicht gleichzeitig für Amino stehen dürfen.

3. Verfahren zur Herstellung und substituierten Malonsäurederivaten der allgemeinen Formel

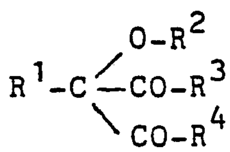

(I)

wobei

$R^1$ = ein- bis dreifach, gleich oder verschieden in 3,4,5-Stellung durch Halogen substituiertes Phenyl

$R^2$ = Wasserstoff, $C_{1\text{-}4}$-Alkyl, gegebenenfalls halogensubstituiertes $C_{1\text{-}4}$-Alkylaminosulfonyl, $C_{1\text{-}4}$-Alkylcarbonyl, gegebenenfalls durch Halogen substituiertes Phenylcarbonyl, Phenylsulfenyl, gegebenenfalls halogensubstituiertes $C_{1\text{-}4}$-Alkylsulfenyl, Trialkylsilyl oder Rest der Formel

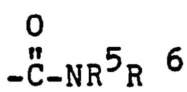

wobei $R^5$ für Wasserstoff steht und $R^6$ für gegebenenfalls durch Halogen substituiertes Phenyl oder Phenylcarbonyl oder für $C_{1\text{-}4}$-Alkyl oder $C_{1\text{-}4}$-Alkylcarbonyl steht,

$R^3$ und $R^4$ oder verschieden sind und für Amino, Trimethylsilylamino, Formylamino, $C_1$-$C_4$-Alkylcarbonylamino, Alkylamino, Trialkylsilylalkylamino stehen, und wobei, falls $R^2$ für Wasserstoff steht, $R^3$ und $R^4$ nicht gleichzeitig für Amino stehen dürfen,

dadurch gekennzeichnet, daß man Verbindungen der Formel

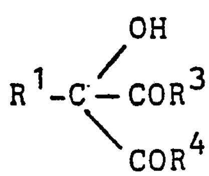

(II)

$a_1$) mit Verbindungen der Formel III

$R^{10}$-Y (III)

wobei

Y für Halogen oder -O-$SO_2$-O-$C_{1\text{-}4}$-Alkyl steht,

$R^{10}$ für $C_{1\text{-}4}$-Alkyl steht,

umsetzt oder

$a_2$) mit Verbindungen der Formel IV

$R^{11}$-Z (IV)

wobei

Z für Halogen, $C_{1-4}$-Alkylcarbonyloxy, CN, steht,
$R^{11}$ für $C_{1-4}$-Alkylcarbonyl, gegebenenfalls durch Halogen substituiertes Phenylcarbonyl, $C_{1-4}$-Alkylaminosulfonyl steht sowie für
$-CONR^5R^6$ steht,
wobei
$R^5$ und $R^6$ die oben angegebene Bedeutung besitzen,
umsetzt oder
$a_3$) mit Verbindungen der Formel V
$R^{12}$-NCO (V)
in welcher
$R^{12}$ für gegebenenfalls durch Halogen substituiertes Phenyl oder Phenylcarbonyl oder für $C_{1-4}$-Alkyl oder $C_{1-4}$-Alkylcarbonyl steht,
umsetzt oder daß man
b) für den Fall, daß $R^2$ für Trialkylsilyl steht, Verbindungen der Formel II mit Trialkylsilyl-Verbindungen der Formel VIII

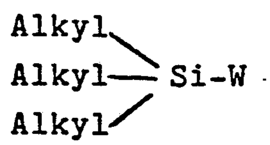

(VIII)

in welcher
W für Halogen bzw. CN, oder für Trialkylsilylamino steht
in annähernd äquimolaren Mengen gegebenenfalls in Gegenwart eines Verdünnungsmittels, gegebenenfalls eines Katalysators und gegebenenfalls in Gegenwart eines Säureakzeptors umsetzt.

4. Verfahren zur Herstellung von Verbindungen der Formel

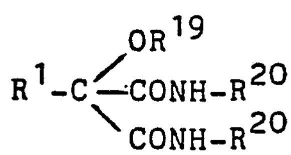

in welcher
$R^1$ die in Anspruch 3 angegebene Bedeutung besitzt,
$R^{19}$ für $C_{1-4}$-Alkylcarbonyl oder für gegebenenfalls durch Halogen substituiertes Phenylcarbonyl steht,
$R^{20}$ für Formyl oder $C_{1-4}$-Alkylcarbonyl steht dadurch gekennzeichnet, daß man Verbindungen der Formel

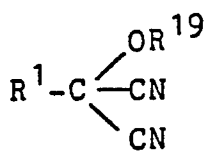

in welcher $R^1$ und $R^{19}$ die oben angegebene Bedeutung haben, mit Säuren der Formel
$R^{20}$-OH
in welcher
$R^{20}$ die oben angegebene Bedeutung hat
oder ihren Anhydriden in Gegenwart anorganischer Mineralsäuren gegebenenfalls unter Zusatz von Wasser umsetzt.

5. Verfahren zur Herstellung von Verbindungen der Formel

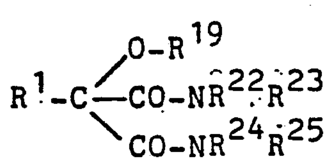

in welcher
$R^1$, $R^{19}$ die in Anspruch 4 angegebene Bedeutung besitzen,
$R^{22}$ für $C_{1-4}$-Alkylcarbonyl
$R^{24}$ für Wasserstoff oder $C_{1-4}$-Alkylcarbonyl steht,

$R^{23}$ für Wasserstoff oder Alkyl steht,
$R^{25}$ für Wasserstoff oder Alkyl steht,
dadurch gekennzeichnet, daß man Verbindungen der Formel

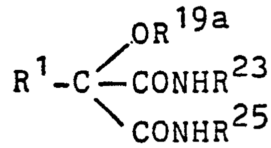

wobei $R^1$, $R^{23}$, $R^{25}$ die oben angegebene Bedeutung besitzen,
$R^{19a}$ für Wasserstoff, $C_{1-4}$-Alkylcarbonyl oder für gegebenenfalls durch Halogen substituiertes Phenylcarbonyl steht, mit Säureanhydriden der allgemeinen Formel

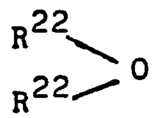

in welcher
$R^{22}$ die oben angegebene Bedeutung hat, umsetzt.

6. Verfahren zur Herstellung von Verbindungen der Formel

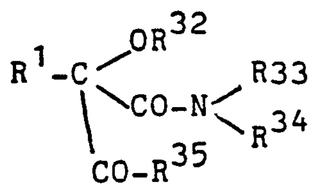

in welcher
$R^1$ die in Anspruch 4 angegebene Bedeutung hat,
$R^{32}$ für Trialkylsilyl, $C_{1-4}$-Alkyl, $C_{1-4}$-Alkylcarbonyl, gegebenenfalls durch Halogen substituiertes Phenylcarbonyl steht,
$R^{33}$ für Wasserstoff steht,
$R^{34}$ für Trimethylsilyl steht,
$R^{35}$ für Trimethylsilylamino, Trialkylsilylalkylamino, Alkylamino steht,
dadurch gekennzeichnet, daß man Verbindungen der Formel

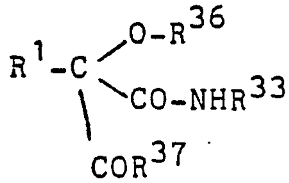

in welcher
$R^1$ und $R^{33}$ die oben engegebene Bedeutung haben und
$R^{36}$ für Wasserstoff, $C_{1-4}$-Alkyl, $C_{1-4}$-Alkylcarbonyl, gegebenenfalls durch Halogen substituiertes Phenylcarbonyl steht,
$R^{37}$ für Amino, Alkylamino steht,
mit Trialkylsilylverbindungen der Formel

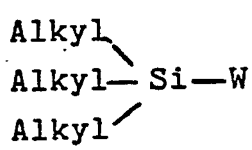

in welcher W die in Anspruch 3 angegebene Bedeutung besitzt, umsetzt.

7. Verfahren zur Herstellung von Verbindungen der Formel XXIV

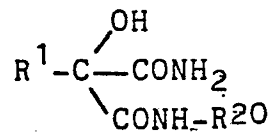

(XXIV)

in welcher
$R^1$ die in Anspruch 1 angegebene Bedeutung hat und
$R^{20}$ für Formyl, Alkylcarbonyl oder gegebenenfalls durch Halogen substituiertes Phenylcarbonyl steht, dadurch gekennzeichnet, daß man Verbindungen der Formel XXV

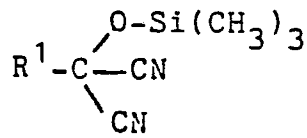

(XXV)

in welcher
$R^1$ die oben angegebene Bedeutung hat, mit Säuren der Formel XXIII
$R^{20}$-OH (XXIII)
in welcher
$R^{20}$ die oben angegebene Bedeutung hat
oder ihren Anhydriden in Gegenwart von anorganischen Mineralsäuren umsetzt.

## Claims

1. Agents for combating pests, characterised in that they contain substituted malonic acid derivatives of the general formula I

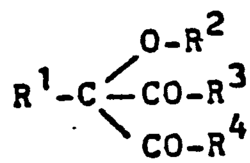

(I)

wherein
$R^1$ = phenyl which is mono- to trisubstituted in the 3,4,5-position by identical or different halogen substituents,
$R^2$ = hydrogen, $C_{1-4}$-alkyl, optionally halogen-substituted $C_{1-4}$-alkyl, aminosulphonyl, $C_{1-4}$-alkylcarbonyl, optionally halogen-substituted phenylcarbonyl, phenylsulphenyl, optionally halogen-substituted $C_{1-4}$-alkylsulphenyl, trialkylsilyl or a radical of the formula

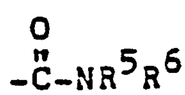

wherein
$R^5$ represents hydrogen and
$R^6$ represents optionally halogen-substituted phenyl or phenylcarbonyl or $C_{1-4}$-alkyl or $C_{1-4}$-alkylcarbonyl,
$R^3$ and $R^4$ are identical or different and represent amino, trimethylsilylamino, formylamino, $C_1$-$C_4$-alkylcarbonylamino, alkylamino, or trialkylsilyl-alkylamino, and, if $R^2$ represents hydrogen, $R^3$ and $R^4$ may not simultaneously represent amino.

2. Substituted malonic acid derivatives of the general formula

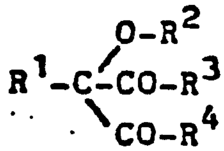

(I)

wherein

$R^1$ = phenyl which is mono- to trisubstituted in the 3,4,5-position by identical or different halogen substituents,

$R^2$ = hydrogen, $C_{1-4}$-alkyl, optionally halogen-substituted $C_{1-4}$-alkyl-aminosulphonyl, $C_{1-4}$-alkyl-carbonyl, optionally halogen-substituted phenylcarbonyl, phenylsulphenyl, optionally halogen-substituted $C_{1-4}$-alkylsulphenyl, trialkylsilyl or a radical of the formula

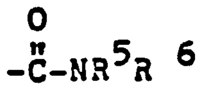

wherein

$R^5$ represents hydrogen and

$R^6$ represents optionally halogen-substituted phenyl or phenylcarbonyl or $C_{1-4}$-alkyl or $C_{1-4}$-alkylcarbonyl,

$R^3$ and $R^4$ are identical or different and represent amino, trimethylsilylamino, formylamino, $C_{1-4}$-alkyl-carbonylamino, alkylamino, or trialkylsilyl-alkylamino, and, if $R^2$ represents hydrogen, $R^3$ and $R^4$ may not simultaneously represent amino.

3. Process for the preparation of substituted malonic acid derivatives of the general formula

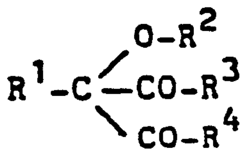

(I)

wherein

$R^1$ = phenyl which is mono- to trisubstituted in the 3,4,5-position by identical or different halogen substituents,

$R^2$ = hydrogen, $C_{1-4}$-alkyl, optionally halogen-substituted $C_{1-4}$-alkylaminosulphonyl, $C_{1-4}$-alkylcarbonyl, optionally halogen-substituted phenylcarbonyl, phenylsulphenyl, optionally halogen-substituted $C_{1-4}$-alkyl-sulphenyl, trialkylsilyl or a radical of the formula

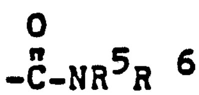

wherein

$R^5$ represents hydrogen and

$R^6$ represents optionally halogen-substituted phenyl or phenylcarbonyl or $C_{1-4}$-alkyl or $C_{1-4}$-alkylcarbonyl,

$R^3$ and $R^4$ are identical or different and represent amino, trimethylsilylamino, formylamino, $C_1$-$C_4$-alkyl-carbonylamino, alkylamino, or trialkylsilylalkylamino, and, if $R^2$ represents hydrogen, $R^3$ and $R^4$ may not simultaneously represent amino,

characterised in that compounds of the formula

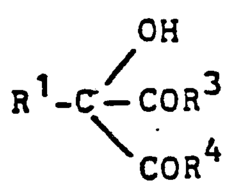

(II)

$a_1$) are reacted with compounds of the formula III

$R^{10}$-Y (III)

wherein

Y represents halogen or $-O-SO_2-O-C_{1-4}$-alkyl, and

$R^{10}$ represents $C_{1-4}$-alkyl, or

$a_2$) are reacted with compounds of the formula IV

$R^{11}$-Z (IV)

wherein

Z represents halogen, $C_{1-4}$-alkylcarbonyloxy, or CN, $R^{11}$ represents $C_{1-4}$-alkylcarbonyl, optionally halogen-substituted phenylcarbonyl, $C_{1-4}$-alkylaminosulphonyl or

$CONR^5R^6$

wherein

$R^5$ and $R^6$ have the abovementioned meaning, or

$a_3$) are reacted with compounds of the formula V

$R^{12}$-NCO (V)

in which

$R^{12}$ represents optionally halogen-substituted phenyl or phenylcarbonyl or $C_{1-4}$-alkyl or $C_{1-4}$-alkylcarbonyl,

or in that

b) in the case where $R^2$ represents trialkylsilyl, compounds of the formula II are reacted with trialkylsilyl compounds of the formula VIII

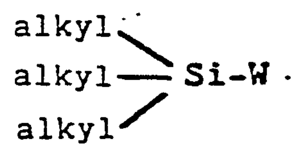

(VIII)

in which

W represents halogen or CN, or trialkylsilylamino, in approximately equimolar quantities, if appropriate in the presence of a diluent, if appropriate a catalyst and if appropriate in the presence of an acid acceptor.

4. Process for the preparation of compounds of the formula

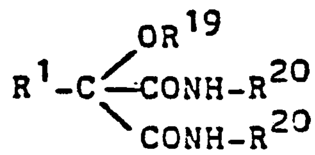

in which

$R^1$ has the meaning given in Claim 3,

$R^{19}$ represents $C_{1-4}$-alkylcarbonyl or optionally halogen-substituted phenylcarbonyl, and

$R^{20}$ represents formyl or $C_{1-4}$-alkylcarbonyl, characterised in that compounds of the formula

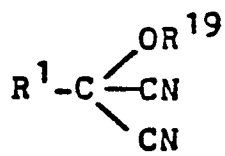

in which

$R^1$ and $R^{19}$ have the abovementioned meaning, are reacted with acids of the formula

$R^{20}$-OH

in which

$R^{20}$ has the abovementioned meaning, or their anhydrides, in the presence of inorganic mineral acids, if appropriate with the addition of water.

5. Process for the preparation of compounds of the formula

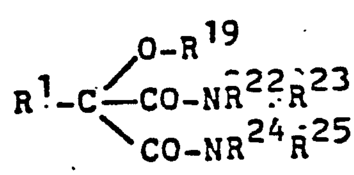

in which

$R^1$ and $R^{19}$ have the meaning given in Claim 4,

$R^{22}$ represents $C_{1-4}$-alkylcarbonyl,

$R^{24}$ represents hydrogen or $C_{1-4}$-alkylcarbonyl,

$R^{23}$ represents hydrogen or alkyl and

$R^{25}$ represents hydrogen or alkyl,
characterised in that compounds of the formula

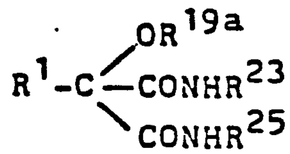

wherein
$R^1$, $R^{23}$ and $R^{25}$ have the abovementioned meaning,
$R^{19a}$ represents hydrogen, $C_{1-4}$-alkylcarbonyl or optionally halogen-substituted phenylcarbonyl, are reacted with acid anhydrides of the general formula

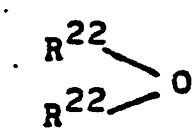

in which
$R^{22}$ has the abovementioned meaning.
6. Process for the preparation of compounds of the formula

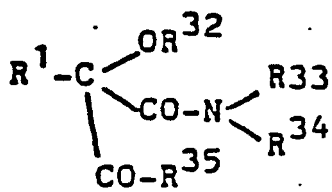

in which
$R^1$ has the meaning given in Claim 4,
$R^{32}$ represents trialkylsilyl, $C_{1-4}$-alkyl, $C_{1-4}$-alkyl-carbonyl, or optionally halogen-substituted phenylcarbonyl,
$R^{33}$ represents hydrogen,
$R^{34}$ represents trimethylsilyl,
$R^{35}$ represents trimethylsilylamino, trialkylsilylalkylamino or alkylamino,
characterised in that compounds of the formula

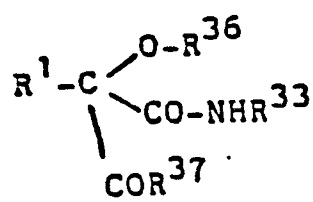

in which
$R^1$ and $R^{33}$ have the abovementioned meaning and
$R^{36}$ represents hydrogen, $C_{1-4}$-alkyl, $C_{1-4}$-alkylcarbonyl or optionally halogen-substituted phenylcarbonyl, and
$R^{37}$ represents amino or alkylamino,
are reacted with trialkylsilyl compounds of the formula

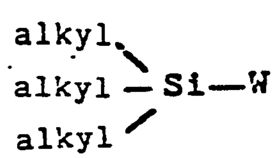

in which
W has the meaning given in Claim 3.
7. Process for the preparation of compounds of the formula XXIV

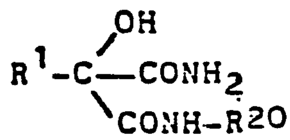

(XXIV)

in which
$R^1$ has the meaning given in Claim 1 and
$R^{20}$ represents formyl alkylcarbonyl or optionally halogen-substituted phenylcarbonyl,
characterised in thas compounds of the formula XXV

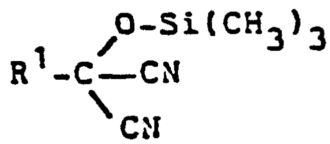

(XXV)

in which
$R^1$ has the abovementioned meaning, are reacted with acids of the formula XXIII
$R^{20}OH$ (XXIII)
in which
$R^{20}$ has the abovementioned meaning,
or their anhydrides in the presence of inorganic mineral acids.

**Revendications**

1. Produits pesticides caractérisés en ce qu'ils contiennent des dérivés substitués de l'acide malonique répondant à la formule générale I

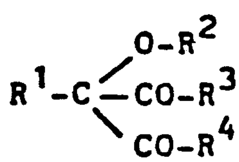

(I)

dans laquelle
$R^1$ = groupe phényle mono- à tri-substitué par des halogènes identiques ou différents en position 3,4,5,
$R^2$ = hydrogène, groupe alkyle en $C_1$-$C_4$, alkyle en $C_1$-$C_4$ éventuellement substitué par des halogènes, aminosulfonyle, (alkyle en $C_1$-$C_4$)-carbonyle, phénylcarbonyle éventuellement substitué par des halogènes, phénylsulfényle, alkylsulfényle en $C_1$-$C_4$ éventuellement substitué par des halogènes, trialkylsilyle ou le groupe de formule

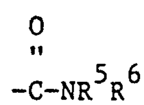

dans lequel
$R^5$ représente l'hydrogène et
$R^6$ un groupe phényle ou phénylcarbonyle éventuellement substitué par des halogènes ou un groupe alkyle en $C_1$-$C_4$ ou (alkyle en $C_1$-$C_4$)-carbonyle,
$R^3$ et $R^4$, ayant des significations identiques ou différentes, représentent chacun un groupe amino, triméthylsilylamino, formylamino, (alkyle en $C_1$-$C_4$)-carbonylamino, alkylamino, trialkylsilyl-alkylamino, avec la restriction que, lorsque $R^2$ représente l'hydrogène, $R^3$ et $R^4$ ne peuvent représenter tous deux un groupe amino.

2. Dérivés substitués de l'acide malonique répondant à la formule générale

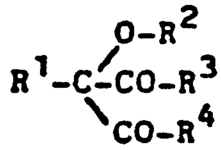

(I)

dans laquelle

$R^1$ = groupe phényle mono- à tri-substitué par des halogènes identiques ou différents en position 3,4,5;

$R^2$ = hydrogène, (alkyle en $C_1$-$C_4$)-aminosulfonyle éventuellement substitué par des halogènes, (alkyle en $C_1$-$C_4$)-carbonyle, phénylcarbonyle éventuellement substitué par des halogènes, phénylsulfényle, alkylsulfényle en $C_1$-$C_4$ éventuellement substitué par des halogènes, trialkylsilyle ou le groupe de formule

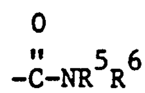

dans lequel

$R^5$ représente l'hydrogène, et

$R^6$ un groupe phényle ou phénylcarbonyle éventuellement substitué par des halogènes ou un groupe alkyle en $C_1$-$C_4$ ou (alkyle en $C_1$-$C_4$)-carbonyle,

$R^3$ et $R^4$, ayant des significations identiques ou différentes, représentent chacun un groupe amino, triméthylsilylamino, formylamino, (alkyle en $C_1$-$C_4$)-carbonylamino, alkylamino, trialkylsilyl-alkylamino, avec la restriction que, lorsque $R^2$ représente l'hydrogène, $R^3$ et $R^4$ ne peuvent représenter tous deux des groupes amino.

3. Procédé de préparation des dérivés substitués de l'acide malonique répondant à la formule générale

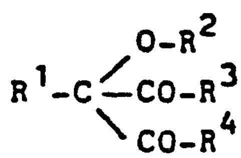

(I)

dans laquelle

$R^1$ = phényle mono- à tri-substitué par des halogènes identiques ou différents en position 3,4,5,

$R^2$ = hydrogène, alkyle en $C_1$-$C_4$, alkylaminosulfonyle en $C_1$-$C_4$ éventuellement substitué par des halogènes, (alkyle en $C_1$-$C_4$)-carbonyle, phénylcarbonyle éventuellement substitué par des halogènes, phénylsulfényle, alkylsulfényle en $C_1$-$C_4$ éventuellement substitué par des halogènes, trialkylsilyle ou le groupe de formule

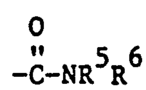

dans lequel

$R^5$ représente l'hydrogène et

$R^6$ un groupe phényle ou phénylcarbonyle éventuellement substitué par des halogènes ou un groupe alkyle en $C_1$-$C_4$ ou (alkyle en $C_1$-$C_4$)-carbonyle,

$R^3$ et $R^4$, ayant des significations identiques ou différentes, représentent chacun un groupe amino, triméthylsilylamino, formylamino, (alkyle en $C_1$-$C_4$)-carbonylamino, alkylamino, trialkylsilylalkylamino avec la restriction que, lorsque $R_2$ représente l'hydrogène, $R^3$ et $R^4$ ne peuvent représenter tous deux des groupes amino, caractérisé en ce que l'on fait réagir des composés de formule

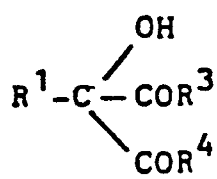

(II)

$a_1$) avec des composés de formule III

$R^{10}$- Y (III)

dans laquelle

Y représente un halogène ou un groupe -O-$SO_2$-O-alkyle en $C_1$-$C_4$,

$R^{10}$ représente un groupe alkyle en $C_1$-$C_4$,
ou bien
$a_2$) avec des composés de formule IV
$R^{11}$-Z (IV)
dans laquelle
Z représente un halogène, un groupe (alkyle en $C_1$-$C_4$)-carbonyloxy, CN,
$R^{11}$ représente un groupe (alkyle en $C_1$-$C_4$)-carbonyle, phénylcarbonyle éventuellement substitué par des halogènes, alkylaminosulfonyle en $C_1$-$C_4$
ou
-$CONR^5R^6$
dans lequel
$R^5$ et $R^6$ ont les significations indiquées ci-dessus, ou bien
$a_3$) avec des composés de formule V
$R^{12}$-NCO (V)
dans laquelle
$R^{12}$ représente un groupe phényle ou phénylcarbonyle éventuellement substitué par des halogènes ou un groupe alkyle en $C_1$-$C_4$ ou (alkyle en $C_1$-$C_4$)-carbonyle, ou bien
b) dans le cas où $R^2$ représente un groupe trialkylsilyle, on fait réagir des composés de formule II avec des composés trialkylsilyliques de formule VIII

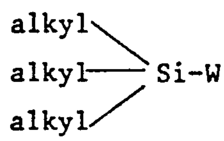
(VIII)

dans laquelle
W représente un halogène ou un groupe CN, ou un groupe trialkylsilylamino, en proportions à peu près équimoléculaires, éventuellement en présence d'un diluant, éventuellement en présence d'un catalyseur et éventuellement en présence d'un accepteur d'acide.
4. Procédé de préparation des composés de formule

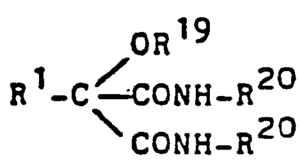

dans laquelle
$R^1$ a les significations indiquées dans la revendication 3,
$R^{19}$ représente un groupe (alkyle en $C_1$-$C_4$)-carbonyle ou un groupe phénylcarbonyle éventuellement substitué par des halogènes,
$R^{20}$ représente un groupe formyle ou (alkyle en $C_1$-$C_4$)-carbonyle, caractérisé en ce que l'on fait réagir des composés de formule

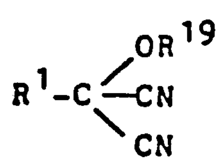

dans laquelle
$R^1$ et $R^{19}$ ont les significations indiquées ci-dessus, avec des acides de formule
$R^{20}$-OH
dans laquelle
$R^{20}$ a les significations indiquées ci-dessus, ou leurs anhydrides, en présence d'acides minéraux, éventuellement avec adjonction d'eau.
5. Procédé de préparation des composés de formule

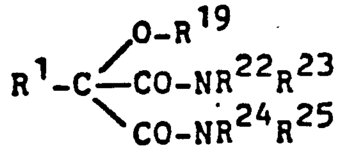

dans laquelle
$R^1$ et $R^{19}$ ont les significations indiquées dans la revendication 4,
$R^{22}$ représente un groupe (alkyle en $C_1$-$C_4$)-carbonyle,
$R^{24}$ représente l'hydrogène ou un groupe (alkyle en $C_1$-$C_4$)-carbonyle,
$R^{23}$ représente l'hydrogène ou un groupe alkyle,
$R^{25}$ représente l'hydrogène ou un groupe alkyle,
caractérisé en ce que l'on fait réagir des composés de formule

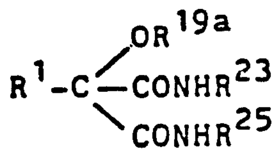

dans laquelle
$R^1$, $R^{23}$, $R^{25}$ ont les significations indiquées ci-dessus,
$R^{19a}$ représente l'hydrogène, un groupe (alkyle en $C_1$-$C_4$)-carbonyle ou phénylcarbonyle éventuellement substitué par des halogènes, avec des anhydrides d'acides de formule générale

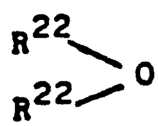

dans laquelle
$R^{22}$ a les significations indiquées ci-dessus.
6. Procédé de préparation des composés de formule

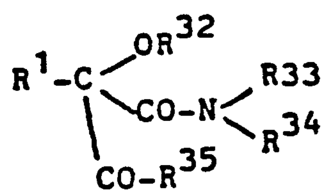

dans laquelle
$R^1$ a les significations indiquées dans la revendication 4,
$R^{32}$ représente un groupe trialkylsilyle, alkyle en $C_1$-$C_4$, (alkyle en $C_1$-$C_4$)-carbonyle, phénylcarbonyle éventeullement subsitué par des halogènes,
$R^{33}$ représente l'hydrogène,
$R^{34}$ représente un groupe triméthylsilyle,
$R^{35}$ représente un groupe triméthylsilylamino, trialkylsilylalkylamino, alkylamino,
caractérisé en ce que l'on fait réagir des composés de formule